(19)

Europäisches
Patentamt
European
Patent Office
Office européen
des brevets

(11) **EP 3 782 589 B1**

(12) **EUROPEAN PATENT SPECIFICATION**

(45) Date of publication and mention
of the grant of the patent:
**17.12.2025 Bulletin 2025/51**

(21) Application number: **19788626.0**

(22) Date of filing: **17.04.2019**

(51) International Patent Classification (IPC):
***A61F 2/66*** (2006.01)

(52) Cooperative Patent Classification (CPC):
**A61F 2/60; A61F 2/66;** A61F 2002/607;
A61F 2002/665; A61F 2002/6678

(86) International application number:
**PCT/JP2019/016546**

(87) International publication number:
**WO 2019/203289 (24.10.2019 Gazette 2019/43)**

(54) **SOLE FOR SPORTS ARTIFICIAL FOOT**

SOHLE FÜR KÜNSTLICHEN SPORTFUSS

SEMELLE POUR PIED ARTIFICIEL DE SPORT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.04.2018 JP 2018079459**

(43) Date of publication of application:
**24.02.2021 Bulletin 2021/08**

(73) Proprietor: **Bridgestone Corporation**
**Chuo-Ku**
**Tokyo 104-8340 (JP)**

(72) Inventors:
• **ITOI, Dyta**
**Tokyo 104-8340 (JP)**
• **SAHASHI, Kohei**
**Tokyo 104-8340 (JP)**

(74) Representative: **Marks & Clerk LLP**
**15 Fetter Lane**
**London EC4A 1BW (GB)**

(56) References cited:
WO-A2-2010/009475          JP-A- 2008 093 016
US-A- 4 607 440             US-A- 5 048 203
US-A1- 2016 045 337         US-A1- 2017 281 371
US-B1- 8 535 390            US-B1- 8 535 390
US-B2- 9 629 415

EP 3 782 589 B1

## Description

TECHNICAL FIELD

The present disclosure relates to a sole attached to a ground contact region of an athletic prosthetic leg, in particular, to a sole of an athletic prosthetic leg which inhibits slip of the prosthetic leg during a competition.

BACKGROUND

Conventionally, a prosthetic leg for a competition (hereinafter, it is referred to as an athletic prosthetic leg or simply referred to as a prosthetic leg) having a leaf-spring-like leg portion which extends via a curved portion to a side of a toe and in which a ground contact region extends from the toe to a side of the curved portion in an arc has been well-known. In such an athletic prosthetic leg having the leaf-spring-like leg portion, generally, a sole which abuts a road surface is attached to a bottom surface of the ground contact region.

For example, Patent Literatures 1 to 3 illustrate a sole which is attached to a lower surface of a curved leaf-spring-like athletic prosthetic leg to correspond to sporting events such as jogging or running. In other words, Patent Literature 1 discloses a sole to which a spike is attached at a lower surface of the sole contacting a road surface or a sole provided with a number of outsole portions each having a hexagonal contact patch. Patent Literature 4 illustrates a sole attached to a lower surface of a curved leaf-spring-like prosthetic leg. Patent Literature 5 illustrates sole structures for footwear articles.

CITATION LIST

Patent Literature

PTL 1: Japanese Patent Laid-Open No. 2016-150189
PTL 2: US 8 535 390 B1
PTL 3: US 2016/045337 A1
PTL 4: US 2017/281371 A1
PTL 5: US 9 629 415 B2

SUMMARY

(Technical Problem)

However, in the sole illustrated in Patent Literature 1, due to early abrasion of a part of the sole, especially a portion at a side of a toe, a service life of the sole itself has been made shorter.

Also, in the sole illustrated in Patent Literature 1, inhibiting slip of the prosthetic leg, that is, anti-slip property is not at all considered. For example, running on a wet road surface is required in a case of a competition in rainfall etc. At that time, when a water film exists on the road surface, the water film is interposed between a bottom surface of the sole and the road surface while hindering ground contact of the bottom surface, resulting that slip is caused. Especially, on a road with a low coefficient friction μ such as asphalt and a stone pavement, there has been a case where a wearer of the prosthetic leg hesitates further acceleration. Accordingly, a sole having a high anti-slip property has been required for the wearer of the prosthetic leg to satisfactorily exert his running skill as athletes.

An object of the present disclosure is to provide a sole of athletic prosthetic leg achieving both wear resistance performance and anti-slip property.

(Solution to Problem)

The inventor earnestly studied means to solve the problem. In other words, while a bottom surface of an athletic prosthetic leg has been reviewed in detail, the inventor newly found that an especially severe abrasion tends to occur at a portion at a side of a toe of the bottom surface of the sole. Moreover, the inventor conceived that anti-slip property can be improved by providing recesses and protrusions at the bottom surface of the sole, and completed the present disclosure.

The invention is set out in the appended set of claims.

(Advantageous Effect)

Due to the present disclosure, a sole of an athletic prosthetic leg achieving both wear performance resistance and anti-slip property can be provided. By attaching this sole to the athletic prosthetic leg, an athlete's skill can be satisfactorily exerted and a long service life of the sole can be achieved.

BRIEF DESCRIPTION OF THE DRAWINGS

The embodiment falling under the claimed invention is the second embodiment described in figures 6-10C. The first embodiment described in figures 1-5 is considered useful for understanding the invention. In the accompanying drawings:

FIG. 1 is a side view of an athletic prosthetic leg to which a sole according to a first embodiment of the present disclosure is attached;
FIG. 2A is a drawing for explaining in stages movement of a leg portion and a ground contact form in a case where the athletic prosthetic leg is worn and a wearer executes straight running;
FIG. 2B is a drawing for explaining in stages the movement of the leg portion and the ground contact form in a case where the athletic prosthetic leg is worn and the wearer executes straight running;

FIG. 2C is a drawing for explaining in stages the movement of the leg portion and the ground contact form in a case where the athletic prosthetic leg is worn and the wearer executes straight running;

FIG. 2D is a drawing for explaining in stages the movement of the leg portion and the ground contact form in a case where the athletic prosthetic leg is worn and the wearer executes straight running;

FIG. 3 is a drawing which illustrates a pattern of a sole bottom surface of the sole of the athletic prosthetic leg according to the first embodiment;

FIG. 4A is a drawing illustrating variation of the shape of a sipe;

FIG. 4B is a drawing illustrating variation of the shape of a sipe;

FIG. 4C is a drawing illustrating variation of the shape of a sipe;

FIG. 5 is a drawing illustrating a portion Q of the sole bottom surface of FIG. 1;

FIG. 6 is a drawing which illustrates a pattern of a sole bottom surface of a sole of an athletic prosthetic leg according to a second embodiment;

FIG. 7 is a drawing illustrating one shape of a width direction land portion of FIG. 6;

FIG. 8 is a drawing illustrating one shape of the width direction land portion of FIG. 6;

FIG. 9A is a drawing for explaining a mutual arrangement in which an arrangement phase of line-symmetric sipe pairs is synchronized with a phase of waves of a width direction land portion;

FIG. 9B is a drawing for explaining a mutual arrangement in which the arrangement phase of the line-symmetric sipe pairs is different from the phase of the waves of the width direction land portion;

FIG. 10A is a drawing illustrating variation of a pair of sipes;

FIG. 10B is a drawing illustrating variation of the pair of sipes; and

FIG. 10C is a drawing illustrating variation of the pair of sipes.

DETAILED DESCRIPTION

Hereinafter, with reference to the drawings, a sole of an athletic prosthetic leg of the present disclosure (hereinafter, it is also referred to as a sole) will be explained in detail with illustration of embodiments thereof.

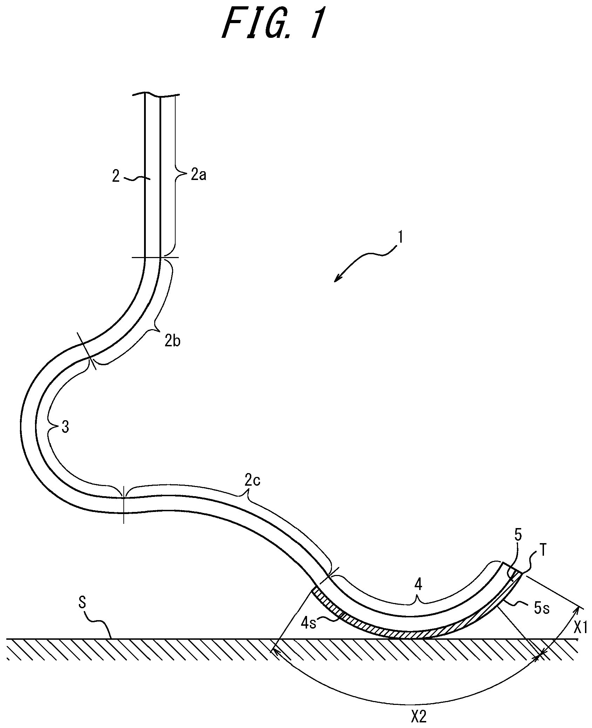

FIG. 1 is a side view of an athletic prosthetic leg 1 to which a sole 5 according to a first embodiment of the present disclosure is attached. The athletic prosthetic leg 1 has a leaf-spring-like leg portion 2, and the sole 5 is attached to a ground contact region at its tip side. Additionally, while illustration is omitted, a base end portion of the leg portion 2 is connected to a socket via an adapter and the socket houses a stump of a wearer's leg, whereby the wearer can wear the prosthetic leg. The adapter and the socket which correspond to the position of the stump of the leg, such as an above-knee prosthesis and a below-knee prosthesis, are used. FIG. 1 illustrates the leg portion 2 and the sole 5 in a standing state of the wearer who wears the athletic prosthetic leg 1.

Hereinafter, in this embodiment, in a height direction of the athletic prosthetic leg, a side where the leg portion 2 is connected to the adapter is referred to as a connection side, and a side where the leg portion 2 contacts a road surface S is referred to as a ground contact side. Also, a toe T of the athletic prosthetic leg 1 refers to a point at the forefront as a termination of the leg portion 2 extending from the connection side. Further, a direction extending from the toe T in parallel with the road surface S is referred to as a leg portion front-rear direction Y. Further, a widthwise direction of the leg portion 2 is referred to as a width direction W.

In this embodiment, the leg portion 2 of the athletic prosthetic leg 1 has a plate-like extending shape to the side of the toe T via at least one curved portion, in the illustrated example, one curved portion 3. In FIG. 1, the leg portion 2 is constituted by, in the order from the connecting side to the ground contact side, a straight portion 2a, a curved portion 2b which is convex to the side of the toe T, the curved portion 3 which is convex to a rear side in the leg portion front-rear direction Y, a curved portion 2c which is concave to the ground contact side and a ground contact portion 4 which is convex to the ground contact side to extend to the side of the toe T in an arc.

Additionally, although the material of the leg portion 2 is not limited, from a viewpoint of strength and weight saving, fiber reinforced plastic etc. is preferably used.

The ground contact portion 4 includes a ground contact region 4s extending from the toe T to the side of the curved portion 3 in an arc at the ground contact side, and the sole 5 is attached to the ground contact region 4s. The ground contact region 4s refers to the entire region abutting the road surface S when the wearer who wears the athletic prosthetic leg 1 executes straight running movement, and in a state that sole 5 is attached, the ground contact region 4s abuts the road surface S via the sole 5.

The sole 5 has a shape conforming to an extending shape of the ground contact region 4s. Also, the ground contact side of the sole 5 is a bottom surface 5s. As illustrated in FIG. 1, the bottom surface 5s has a shape in which an arc X1 and an arc X2 are continued from the toe T side to the curved portion 3 side. While the arc X1 and the arc X2 have a different radius of curvature to each other in this embodiment, they may include the same radius of curvature.

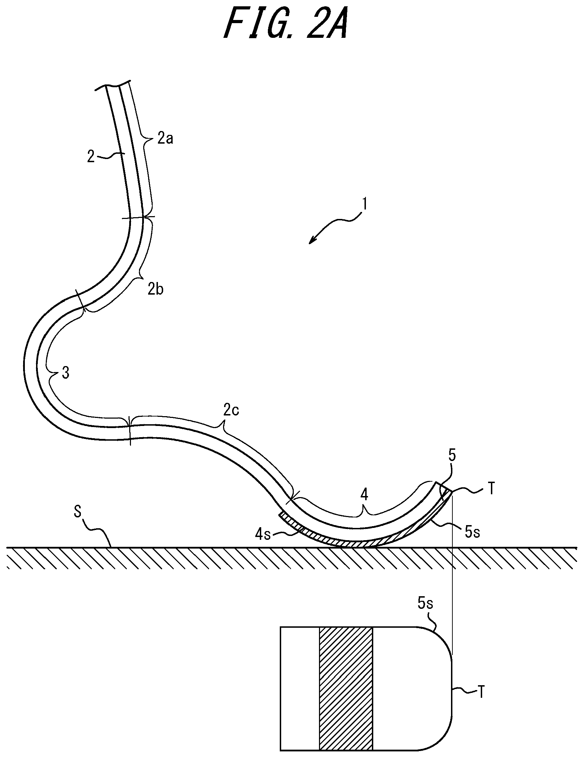

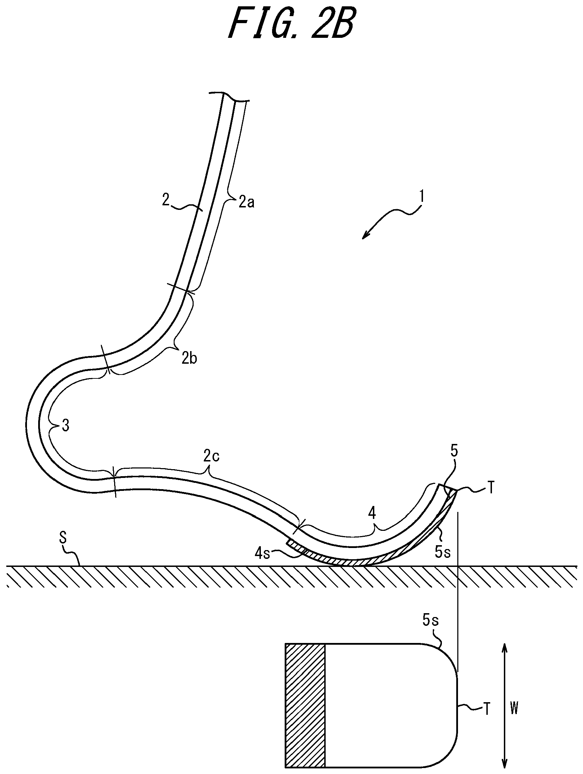

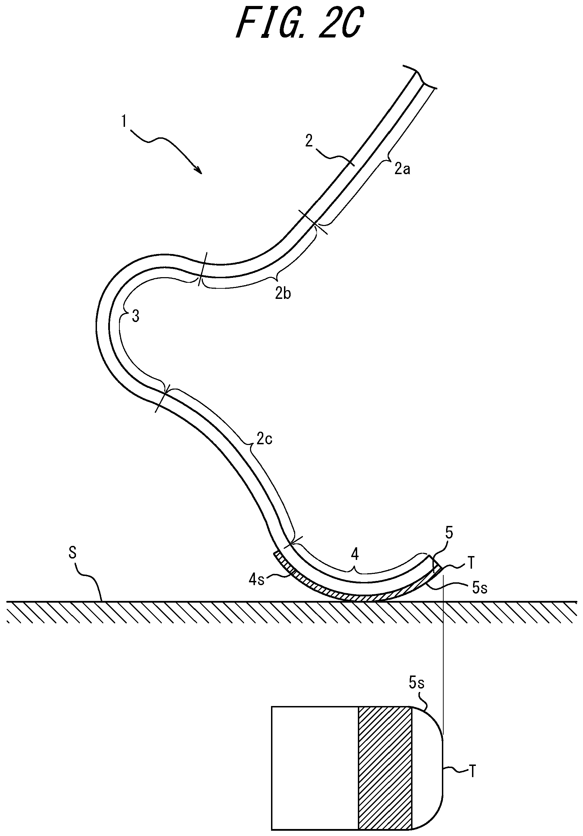

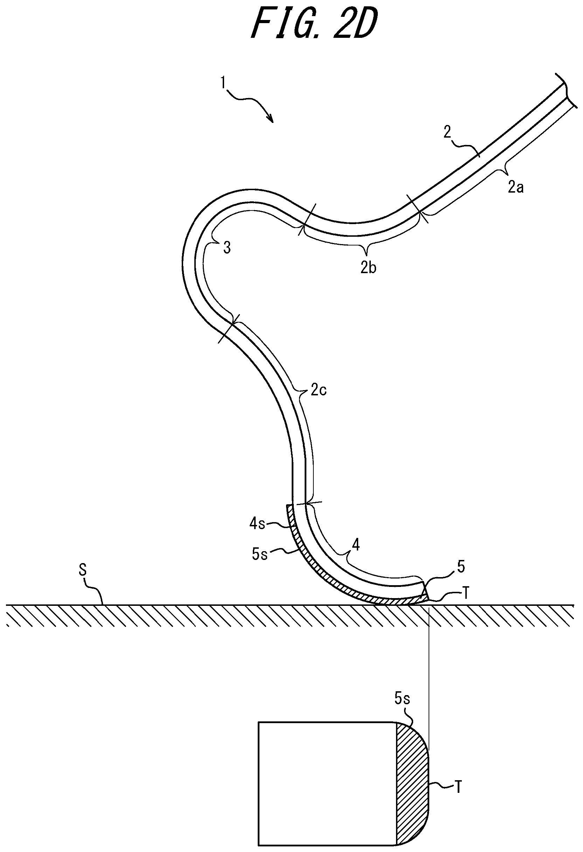

A pattern of the bottom surface 5s of the sole of the athletic prosthetic leg according to the first embodiment of the present disclosure is based on a finding related to a ground contact form obtained from an experiment which will be described later. Accordingly, an experiment result of the ground contact form of the bottom surface 5s as described above will be explained below using FIGS. 2A, 2B, 2C and 2D. FIGS. 2A, 2B, 2C and 2D are drawings for explaining in stages movement of the leg portion 2 and the ground contact form of the bottom surface 5s when the wearer who wears the athletic prosthetic leg 1 executes straight running. In each drawing, an upper portion is a side view of the leg portion 2 and the sole 5, and a lower portion illustrates a transition of the ground contact form of the bottom surface 5s when the wearer who wears the athletic prosthetic leg 1 executes straight running.

In other words, FIG. 2A illustrates a state that the wearer lowers the raised athletic prosthetic leg 1 to the road surface S and the entire weight is loaded on the athletic prosthetic leg 1. As illustrated in the lower portion of the drawing, at an initial stage of lowering the prosthetic leg 1 on the road surface S, a ground contact region of the bottom surface 5s is located to be adjacent to a center portion of the bottom surface, which is a region spaced from both the curved portion 3 and a side of the toe T in the leg portion front-rear direction.

FIG. 2B illustrates a state that the wearer steps forward while the entire weight remains to be loaded on the athletic prosthetic leg 1. In a case of running of a healthy person, such a step form is generally applied that ground contact is sequentially executed from a heel side which firstly contacts the ground toward a toe side of a shoe sole, while in the athletic prosthetic leg 1, the ground contact region is moved to the side of the curved portion 3 from a portion which firstly contacts the ground.

FIG. 2C illustrates a state that the wearer starts a kick-out movement of the athletic prosthetic leg 1 by shaking an opposite leg from the leg wearing the athletic prosthetic leg 1 forward. Entering into this kick-out movement, the athletic prosthetic leg 1 contacts the ground at a region at the side of the toe T of the bottom surface 5s from the regions illustrated in FIGS. 2A and 2B.

FIG. 2D illustrates a state that the wearer is in a final stage of kicking out the athletic prosthetic leg 1 just before separating from the road surface S. To kick out from the toe T of the bottom surface 5s, ground contact is executed further at the side of the toe T than in FIG. 2C.

The bottom surface 5s based on the experiment result illustrated in FIGS. 2A, 2B, 2C and 2D will be explained below.

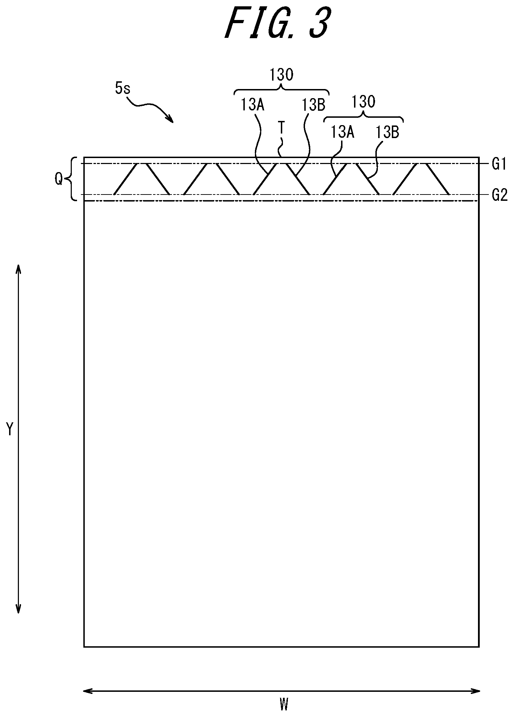

As illustrated in FIG. 3, the bottom surface 5s includes at least one, ten in the illustrated example, sipes 13A and 13B linearly extending at a portion Q corresponding to the arc X1 which continues at a constant radius of curvature from the toe. The sipes 13A and 13B terminate in a land portion of the portion Q of the bottom surface 5s. Here, in this embodiment, terminating in the bottom surface 5s means that the sipes 13A and 13B are not open at an end edge of the sole 5, which refers to a sipe having both ends which terminate in the portion Q of the bottom surface 5s.

In other words, as illustrated in FIG. 2D, the portion Q is a region where the wearer shakes an opposite leg from a leg wearing the athletic prosthetic leg 1 forward to execute the kick-out movement of the athletic prosthetic leg 1. The portion Q and a portion adjacent thereto sequentially contact the ground toward the toe T, and the wearer presses the road surface S by the bottom surface 5s to slidingly contact the ground, so that these portions are a region which easily develops abrasion in particular. Especially, the portion Q finally contacts the ground when the wearer wearing the athletic prosthetic leg 1 executes the kick-out movement, so that a severer abrasion has been inclined to occur. Further, in this region, it is vital that slip prevention is maintained in addition to improving wear resistance performance. Moreover, the sipes 13A and 13B with a groove width of less than 1 mm terminating in the bottom surface 5s are formed, and edge components are distributed without deteriorating rigidity of the bottom surface 5s, so that both wear resistance performance and anti-slip property can be achieved. Further, according to the configuration of the sipes 13A and 13B, occurrence of cracking at the portion Q can also be inhibited.

As illustrated in FIG. 3, the sipes 13A and 13B are preferably linear without any bent portion. In other words, with the sipe being linear, not in a bending shape by a bent portion, occurrence of cracking starting from the bent portion can be inhibited.

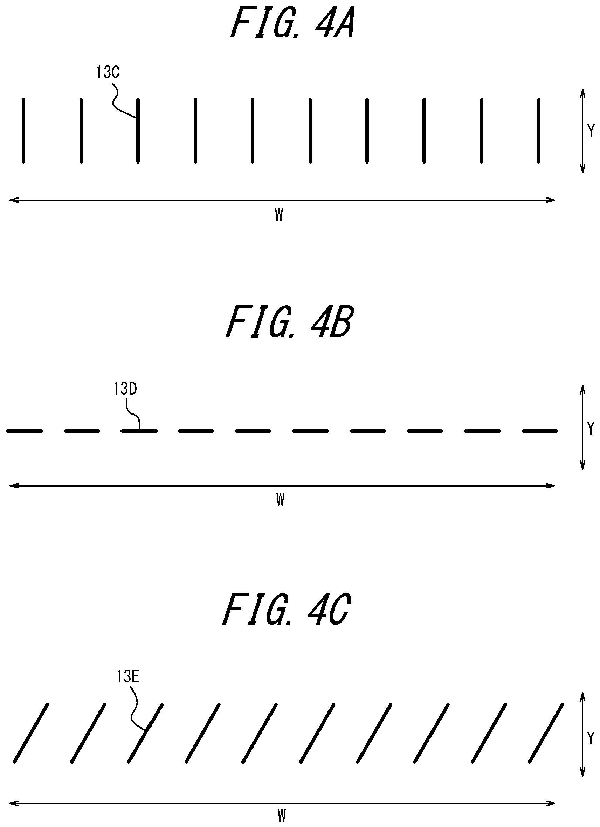

Additionally, the sipes 13A and 13B are not limited to the shape shown in FIG. 3, and may have the shape illustrated in FIGS. 4A, 4B and 4C. A sipe 13C illustrated in FIG. 4A is linear along the leg portion front-rear direction Y. Also, a sipe 13D illustrated in FIG. 4B is linear along the width direction W. Further, a sipe 13E illustrated in FIG. 4C has a shape inclined from the leg portion front-rear direction Y.

Also, as illustrated in FIG. 3, a plurality of sipes 13A and 13B are preferably used. By using the plurality of sipes 13A and 13B, sufficient drainage performance can be achieved. On the other hand, from a viewpoint of inhibiting rigidity deterioration, the total number of the sipes 13A to 13E formed at the portion Q is preferably less than 20.

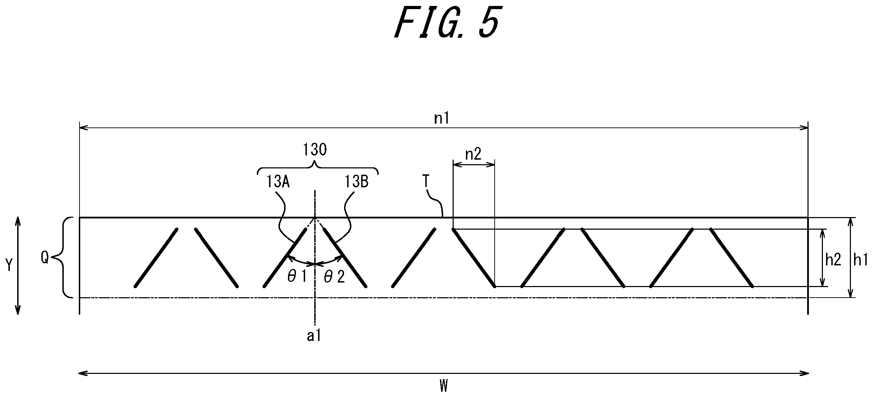

Regarding the shape of the sipes 13A and 13B, an explanation will be made in more detail with reference to FIG. 5.

As illustrated in FIG. 5, the plurality of sipes 13A and 13B preferably include at least one line-symmetric sipe pair 130 constituted by two line-symmetric sipes in a line-symmetric relation with a line along the leg portion front-rear direction Y being set to an axis of symmetry $a1$, and in the illustrated example, five pairs are included. By including the line-symmetric sipe pair 130, an edge function can be evenly applied to a toe portion of the bottom surface 5s.

Also, from a viewpoint of exerting a fully edge function to various input of force, as illustrated in FIG. 5, the sipes 13A and 13B preferably extend in a direction inclined to the axis of symmetry $a1$. More specifically, an inclination angle $\theta1$ or $\theta2$ to the axis of symmetry $a1$ of each of the sipes 13A and 13B is more preferably 20° to 50°.

Also, the line-symmetric sipe pair 130 preferably has an arrangement that each of the sipes 13A and 13B constituting the line-symmetric sipe pair 130 extends in a direction converging to a side of the toe T of the axis of symmetry a1. With this configuration, since the sipe 13A and the sipe 13B extend in different directions, a land portion adjacent to each sipe produces a different response to an input of force from a certain direction, thereby inhibiting deformation as the entire Q portion. Further, an edge function can be efficiently exerted along the direction that the sipes 13A and 13B converge.

Also, such a relation is preferably satisfied that a length h2 of the sipes 13A and 13B along the leg portion front-rear direction Y, to a length h1 of the portion Q in the leg portion front-rear direction Y, has a ratio h2/h1 of 0.4 to 0.9, while a length n2 along the width direction W, to a length n1 of the portion Q in the width direction W, has a ratio of n2/n1 of 0.075 to 0.095. With this configuration, the drainage performance and the wear resistance performance can be balanced.

Additionally, in this embodiment, an aspect of recesses and protrusions formed at regions other than the portion Q of the bottom surface 5s is not especially limited.

Further, based on the experiment result illustrated in 2A, 2B, 2C and 2D, the inventor obtained the finding that separating functions of a bottom surface 50s in accordance with the transition of the ground contact region is advantageous to property improvement of the sole of the prosthetic leg, and conceived the following groove pattern.

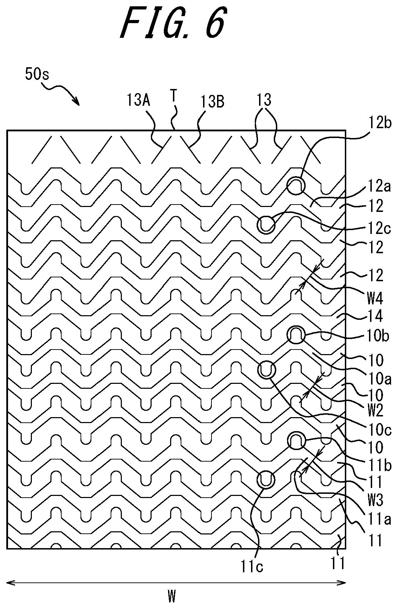

As illustrated in FIG. 6, the bottom surface 50s has an array of the sipe pairs in which the line-symmetric sipe pairs 130 are arranged at the portion Q in the width direction W and, at the side of the curved portion 3 from the portion Q, a plurality of width direction land portions extending to be wavy-shaped in the width direction W defined by a plurality of width direction grooves extending in the width direction W. In other words, in the groove pattern illustrated in FIG. 6, at the portion Q of the bottom surface 50s, five line-symmetric sipe pairs 130 are arranged in the width direction W. Further, in this groove pattern, at the side of the curved portion 3 from the portion Q, a plurality of width direction land portions 12, 14, 10 and 11 extending to be wavy-shaped in the width direction W defined by the plurality of width direction grooves extending in the width direction W are sequentially arranged.

The wave described herein refers to, not merely a sinusoidal wave, one in which a substantially identical shape executes phase variation with a substantially identical cycle such as zigzag and recesses and protrusions, and among these, a sinusoidal wave or a zig-zag shape with an identical phase is preferable.

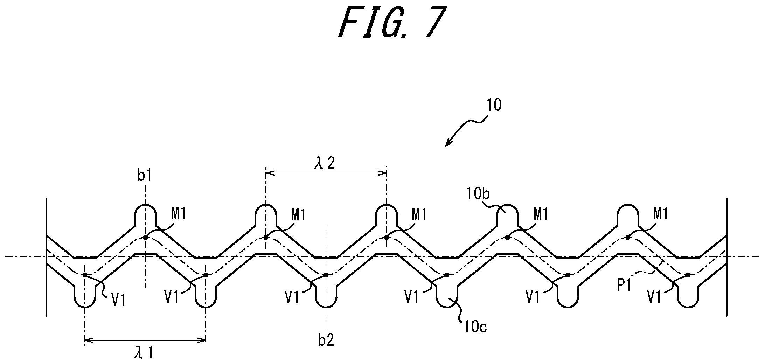

The shape of the width direction land portions 10, 11, 12 and 14 will be explained with reference to FIGS. 6 and 7.

As illustrated in FIG. 7, the width direction land portion 10 is a land portion extending with a predetermined width along a wavy line P1 which is wavy-shaped as described above. By allowing the width direction land portion 10 to be wavy-shaped in this way, an edge component in the width direction W increases and also an edge component in a front-rear direction can be applied, and comprehensively, a higher edge effect can be obtained. In other words, even if a direction of the input of force is shifted from the width direction W at the time of running of the wearer of the athletic prosthetic leg 1, the edge effect can be fully exerted.

Additionally, the width direction land portion 10 is wavy-shaped, which means that, as illustrated in FIGS. 6 and 7, a toe side protruding portion 10b and a curved portion side protruding portion 10c which protrude to one side or the other side in the leg portion front-rear direction from a position of a ridge M1 or a valley V1 of the wavy line P1 may be included. Due to the inclusion of the toe side protruding portion 10b and the curved portion side protruding portion 10c, the edge component is further increased and the edge effect in both the leg portion front-rear direction Y and the width direction W can be improved.

Further, the width direction land portion 10 is preferably line-symmetric about a line extending in the leg portion front-rear direction Y through the ridge M1 or the valley V1 of a wave in one wavelength of the wave (from one ridge to another ridge or from one valley to another valley of a wave). As illustrated in FIG. 7, the width direction land portion 10 is line-symmetric about a line b1 extending in the leg portion front-rear direction Y through the ridge M1 of the wavy line P1 in one wavelength λ1 of a wave, and line-symmetric about a line b2 extending in the leg portion front-rear direction Y through the valley V1 in one wavelength λ2 of the wave. With this configuration, even if a direction of the input of force is shifted from the width direction W at the time of running of the wearer of the athletic prosthetic leg 1, the edge effect can be fully exerted.

The width direction land portions 11 and 14 have the shape extending to be wavy-shaped with the same phase as in the width direction land portion 10. Consequently, in the same manner as the width direction land portion 10, the width direction land portions 11 and 14 are land portions extending with a predetermined width along a line which is wavy-shaped following the above-described definition. Additionally, the difference between the width direction land portions 11, 14 and the width direction land portion 10 is as described later, and the other configurations follow the above-described configuration of the width direction land portion 10.

Here, a region where the width direction land portions 10, 11 and 14 are arranged corresponds to the ground contact regions illustrated in FIGS. 2A and 2B, which is the region where the wearer firstly contact the ground and executes a step movement in a state that the entire weight is loaded on the athletic prosthetic leg 1.

Consequently, to maintain balance of the entire body even if the wearer places his entire weight on the athletic prosthetic leg 1, it is vital that the region fully grips the road surface S. Accordingly, by arranging the width direction land portion 10 with an excellent edge effect in the width direction W and the width direction land portions 11, 14 which are similar to the width direction land portion 10, an excellent edge function is applied to this region and a gripping force to the road surface S is fully secured, which provides a high anti-slip property to the region.

Additionally, the width direction land portion 11 has a larger width than the width direction land portion 10. As illustrated in FIG. 2B, in a state of stepping immediately after the ground contact, the ground contact region is changed to the side of the curved portion 3 from a region which firstly contacts the ground, that is, to the opposite side from a direction that the wearer advances. Here, movement of an upper body in which the wearer tries to move forward and a direction of the change of the ground contact region are temporarily opposite, and this is a stage in which a high propulsive force is needed for the kick-out movement at the latter half of the ground contact form as illustrated in FIGS. 2C to 2D. By arranging the width direction land portion 11 having a larger width than the width direction land portion 10 at the side of the curved portion 3 from the width direction land portion 10 to make a land portion ratio higher, rigidity of this arrangement region is increased, and a high propulsive force is achieved such that the step movement is smoothly continued to the kick-out movement.

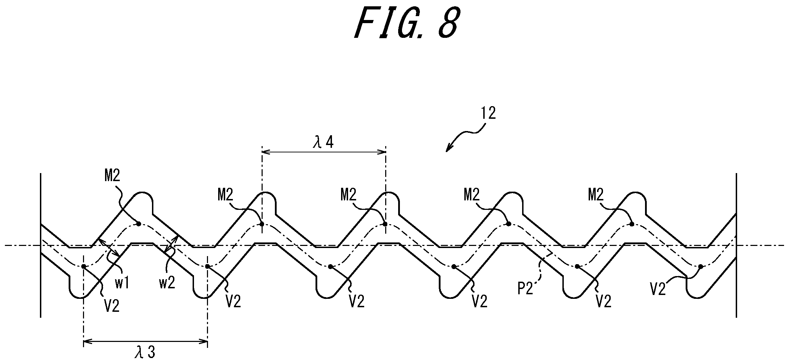

Further, at the side of the toe T of the width direction land portion 14, the width direction land portions 12 are sequentially arranged. A region where the width direction land portions 12 are arranged is the region which contacts the ground when the kick-out movement of the athletic prosthetic leg 1 is started as illustrated in FIG. 2C. Each width direction land portion 12 is the land portion extending to be wavy-shaped in the same manner as the width direction land portion 10, and wherein, especially, a land portion width which is a width in a direction of a normal line of the wavy line is varied. In other words, it is wavy-shaped in which the land portion width in a 1/2 wavelength of a wave (from a ridge to a valley or from a valley to a ridge of the wave) is repeatedly wide and narrow. More specifically, as illustrated in FIG. 8, each width direction land portion 12 is wavy-shaped such that, in a 1/2 wavelength of one wavelength $\lambda 3$ of a wave, that is, in a land portion width w1 from a valley V2 to an adjacent ridge M2 of a wavy line P2 and a land portion width w2 from the ridge M2 to an adjacent valley V2, such a relation is repeated that the land portion width w1 is relatively wide, while the land portion width w2 is relatively narrow. Also, in a 1/2 wavelength of one wavelength $\lambda 4$ of the wave, that is, in a land portion width w2 from a ridge M2 to an adjacent valley V2 of the wavy line P2 and a land portion width w1 from the valley V2 to an adjacent ridge M2, such a relation is repeated that the w1 is relatively wide, while the w2 is relatively narrow. By allowing the land portion width w1 at one side to be wider compared to the land portion width w2 at the other side, land portion rigidity can be improved, and wear resistance performance can be improved. In other words, by allowing the land portion width w2 at the other side to be narrower compared to the one side, slipperiness due to widening of the land portion width can be avoided. Considering comprehensively the above, by appropriately setting the land portion widths w1 and w2, both the drainage performance and the wear resistance performance can be achieved at a high dimension. From such a viewpoint, a ratio w1/w2 of the land portion widths w1 and w2 is preferably 2.0 to 15. More preferably, the land portion widths w1 and w2 satisfy the following numerical range.

$$2.0 \text{ mm} < w1 < 4.0 \text{ mm}$$

$$0.3 \text{ mm} < w2 < 1.0 \text{ mm}$$

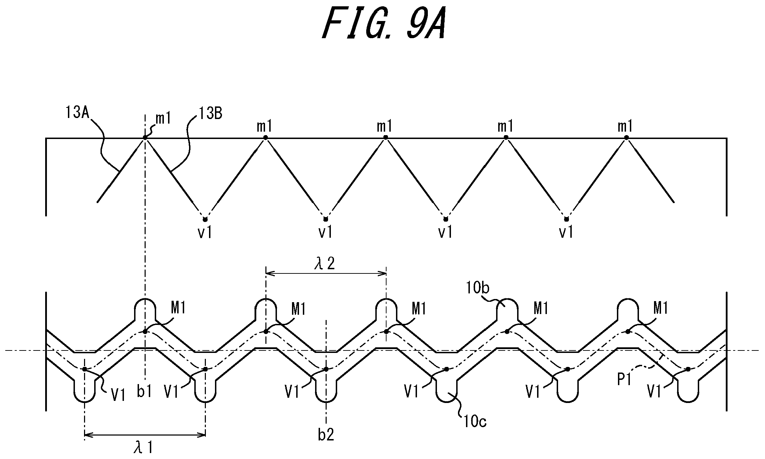

Here, in this embodiment, such a mutual arrangement is preferable that an arrangement phase of the line-symmetric sipe pair 130 is synchronized with a phase of waves of the width direction land portions 10, 11, 12 and 14. As illustrated in FIG. 9A, a plurality of line-symmetric sipe pairs 130, five pairs are illustrated here, are arranged along the width direction W to make an array of the sipe pairs, which has an arrangement along a triangular wave made by connecting, in the width direction W, intersection points of lines virtually extended from both ends of each of the sipes 13A and 13B constituting the array along the shape of each sipe. These intersection points are referred to as a ridge m1 and a valley v1 of the triangular wave. As illustrated in FIG. 9A, a position of the ridge m1 in the width direction W of the triangular wave followed by the array of the plurality of line-symmetric sipe pairs 130 corresponds to a position of the ridge M1 of the wavy-shaped width direction land portion 10, and moreover, a position of the valley v1 in the width direction W corresponds to a position of the valley V1 of the wavy-shaped width direction land portion 10. Additionally, in this embodiment, such a mutual arrangement is presented that the waves of the width direction land portion 10 have the same phase as in the waves in the width direction land portions 11, 12 and 14, and an arrangement phase of the line-symmetric sipe pairs 130 is synchronized with the phase of the waves of the width direction land portions 10, 11, 12 and 14.

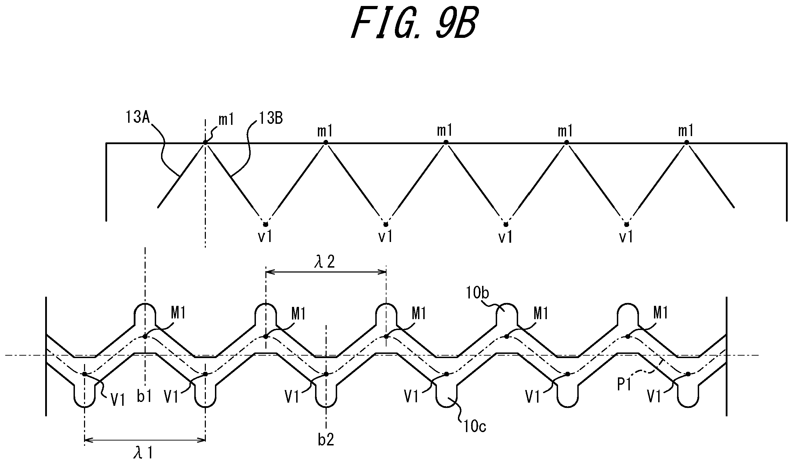

With this configuration, equalization of rigidity distribution at the sole bottom surface 50s can be achieved. In other words, an aspect that the sipes 13A and 13B constituting the line-symmetric sipe pair 130 are convex toward the side of the toe T or the side of the curved portion 3 corresponds to an aspect of convex portions of the wavy-shaped width direction land portions 10, 11, 12 and 14, which prevents deterioration of the rigidity in the sole bottom surface 50s. As illustrated in FIG. 9B, in a case where a phase of the triangular wave followed by the array of the line-symmetric sipe pairs 130 is different from a phase of the width direction land portions 10, 11, 12 and 14, a deformation occurs at a portion where a position of the valley v1 in which the line-symmetric sipe pair 130 is convex corresponds to a position of the ridge M1 of the waves of the width direction land portion 10 in the width direction W, so that the rigidity of the sole bottom surface 50s may be deteriorated. Consequently, as illustrated in FIG. 9A, by allowing the arrangement phase of the line-symmetric sipe pair 130 to correspond to the phase of the wavy line P1 of the waves of the width direction land portion 10, no deformation occurs, which achieves equalization of the rigidity distribution at the bottom surface 50s.

Additionally, the array of the line-symmetric sipe pairs 130 is preferably arranged with even intervals in the width direction W and on a straight line. In other words, as illustrated in FIG. 3, in the array of the line-symmetric sipe pairs 130, a minimum width and a maximum width of a gap between adjacent line-symmetric sipe pairs 130 are uniform, and in the leg portion front-rear direction Y, each end portion at the side of the toe T of the sipes 13A and 13B constituting each sipe pair 130 is arranged on a straight line G1 along the width direction W, while each end portion at the side of the curved portion 3 is arranged on a straight line G2 along the width direction W. With this arrangement, equalization of rigidity distribution at the portion Q can be achieved.

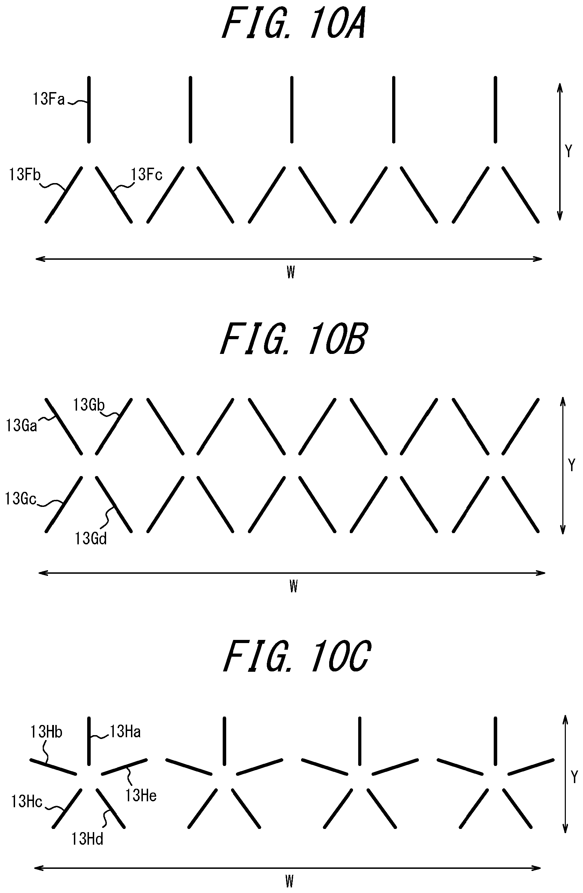

Additionally, while the line-symmetric sipe pair 130 refers to the above-described line-symmetric sipe pair constituted by two sipes in this embodiment, in other embodiments, one sipe pair may be constituted by three or more sipes. When one sipe pair is constituted by three or more sipes, one sipe pair is preferably radially arranged. FIG. 10A is a case where one sipe pair is constituted by three sipes, in which sipes 13Fa, 13Fb, 13Fc are paired and radially arranged. Also, FIG. 10B is a case where one sipe pair is constituted by four sipes, in which sipes 13Ga, 13Gb, 13Gc and 13Gd are paired and radially arranged. Further, FIG. 10C is a case where one sipe pair is constituted by five sipes, in which sipes 13Ha, 13Hb, 13Hc, 13Hd and 13He are paired and radially arranged.

REFERENCE SIGNS LIST

1 athletic prosthetic leg
2 leg portion
2a straight portion
2b, 2c curved portion
3 curved portion
4 ground contact portion
4s ground contact region
5 sole

5s, 50s sole bottom surface
10, 11, 12, 14 land portion
10b toe side protruding portion
10c curved portion side protruding portion
13A, 13B, 13C, 13D, 13E sipe
13Fa, 13Fb, 13Fc sipe
13Ga, 13Gb, 13Gc, 13Gd sipe
13Ha, 13Hb, 13Hc, 13Hd, 13He sipe
130 line-symmetric sipe pair
M1, M2, m1 ridge
V1, V2, v1 valley

## Claims

1. A sole (5) of an athletic prosthetic leg (1), the athletic prosthetic leg (1) having a leaf-spring-like leg portion (2) extending to a side of a toe (T) via at least one curved portion (3), the sole (5) being configured to be attached to a ground contact region (4s) of the athletic prosthetic leg (1), the ground contact region (4s) extending from the toe (T) to a rear side in a leg portion front-rear direction (Y) in a first arc (X1) and a second arc (X2) and being convex to a ground contact side, wherein

the sole (5) includes a bottom surface (5s) having a shape conforming to an extending shape of the ground contact region (4s), and
the bottom surface (5s) includes at least one sipe (13) linearly extending at a first portion (Q) corresponding to the first arc (X1) which continues at a constant radius of curvature from a toe of the bottom surface (5s), and the at least one sipe (13) has a groove width of less than 1 mm, is not open at an end edge of the sole (5) and has both ends which terminate in the first portion (Q) of the bottom surface (5s),
wherein the at least one sipe (13) comprises a plurality of sipes (13),
wherein the plurality of sipes (13) include at least one pair (130) of two line-symmetric sipes (13A, 13B) in a line-symmetric relation with a line along a front-rear direction (Y) of the leg portion (2) being set to an axis of symmetry,
wherein, in the line-symmetric sipe pair (130), each sipe (13A, 13B) extends in a direction inclined to the axis of symmetry,
wherein the bottom surface (5s) has, at the first portion (Q) of the bottom surface (5s), an array of sipe pairs (130) in which a plurality of line-symmetric sipe pairs (130) are arranged in a leg portion width direction (W) and, at a second portion of the bottom surface (5s) extending at the side of the curved portion (3) of the first portion (Q) of the bottom surface (5s), a plurality of width direction land portions (10, 11, 12, 14) extending to be wavy-shaped in the leg portion width direction (W) defined by a plurality of width direction grooves extending in the leg portion width direction (W), and

such a mutual arrangement is achieved that an arrangement phase of each line-symmetric sipe pair (130) is synchronized with a phase of waves of each width direction land portion (10, 11, 12, 14).

2. The sole (5) of the athletic prosthetic leg (1) according to claim 1, wherein the sipe (13) is linear without any bent portion.

3. The sole (5) of the athletic prosthetic leg (1) according to claim 1 or claim 2, wherein an inclination angle θ to the axis of symmetry of each sipe (13) is 20° to 50°.

4. The sole (5) of the athletic prosthetic leg (1) according to any one of claims 1 to 3, wherein, in the line-symmetric sipe pair (130), each sipe (13A, 13B) extends in a direction converging with respect to the axis of symmetry, towards the side of the toe.

5. The sole (5) of the athletic prosthetic leg (1) according to any one of claims 1 to 4, wherein the array of the line-symmetric sipe pairs (130) is arranged in the leg portion width direction (W) at even intervals and in a straight line.

**Patentansprüche**

1. Sohle (5) einer Sportbeinprothese (1), wobei die Sportbeinprothese (1) einen blattfederartigen Beinabschnitt (2) aufweist, der sich über mindestens einen gekrümmten Abschnitt (3) zu einer Seite einer Zehe (T) erstreckt, wobei die Sohle (5) so konfiguriert ist, dass sie an einem Bodenkontaktbereich (4s) der Sportbeinprothese (1) befestigt werden kann, wobei sich der Bodenkontaktbereich (4s) von der Zehe (T) zu einer Rückseite in einer Vorne-Hinten-Richtung (Y) des Beinabschnitts in einem ersten Bogen (X1) und einem zweiten Bogen (X2) erstreckt und zu einer Bodenkontaktseite hin konvex ist, wobei

die Sohle (5) eine Bodenfläche (5s) einschließt, die eine Form aufweist, die sich einer sich erstreckenden Form des Bodenkontaktbereichs (4s) anschmiegt, und
die Bodenfläche (5s) mindestens eine Lamelle (13) einschließt, die sich linear an einem ersten Abschnitt (Q) erstreckt, der dem ersten Bogen (X1) entspricht, der sich mit einem konstanten Krümmungsradius von einer Zehe der Bodenfläche (5s) fortsetzt, und die mindestens eine Lamelle (13) eine Rillenbreite von weniger als 1 mm aufweist, an einer Endkante der Sohle (5) nicht offen ist und beide Enden aufweist, die im ersten Abschnitt (Q) der Bodenfläche (5s) enden,
wobei die mindestens eine Lamelle (13) eine Vielzahl von Lamellen (13) umfasst,
wobei die Vielzahl von Lamellen (13) mindestens ein Paar (130) aus zwei liniensymmetrischen Lamellen (13A, 13B) in einer liniensymmetrischen Beziehung mit einer Linie entlang einer Vorne-Hinten-Richtung (Y) des Beinabschnitts (2) einschließt, die auf eine Symmetrieachse festgelegt ist,
wobei sich in dem liniensymmetrischen Lamellenpaar (130) jede Lamelle (13A, 13B) in einer zur Symmetrieachse geneigten Richtung erstreckt,
wobei die Bodenfläche (5s) am ersten Abschnitt (Q) der Bodenfläche (5s) eine Reihe von Lamellenpaaren (130) aufweist, in der eine Vielzahl von liniensymmetrischen Lamellenpaaren (130) in einer Breitenrichtung (W) des Beinabschnitts angeordnet sind, und an einem zweiten Abschnitt der Bodenfläche (5s), der sich an der Seite des gekrümmten Abschnitts (3) des ersten Abschnitts (Q) der Bodenfläche (5s) erstreckt, eine Vielzahl von in Breitenrichtung verlaufenden Stegabschnitten (10, 11, 12, 14), die sich wellenförmig in der Breitenrichtung (W) des Beinabschnitts erstrecken, die durch eine Vielzahl von in Breitenrichtung verlaufenden Rillen definiert sind, und
eine derartige gegenseitige Anordnung erreicht wird, dass eine Anordnungsphase jedes liniensymmetrischen Lamellenpaars (130) mit einer Phase der Wellen jedes in Breitenrichtung verlaufenden Stegabschnitts (10, 11, 12, 14) synchronisiert ist.

2. Sohle (5) der Sportbeinprothese (1) nach Anspruch 1, wobei die Lamelle (13) geradlinig und ohne gebogenen Abschnitt ist.

3. Sohle (5) der Sportbeinprothese (1) nach Anspruch 1 oder Anspruch 2, wobei ein Neigungswinkel 0 zur Symmetrieachse jeder Lamelle (13) 20° bis 50° beträgt.

4. Sohle (5) der Sportbeinprothese (1) nach einem der Ansprüche 1 bis 3, wobei sich im liniensymmetrischen Lamellenpaar (130) jede Lamelle (13A, 13B) in einer Richtung erstreckt, die in Bezug auf die Symmetrieachse zur Zehenseite hin konvergiert.

5. Sohle (5) der Sportbeinprothese (1) nach einem der Ansprüche 1 bis 4, wobei die Reihe der liniensymmetrischen Lamellenpaare (130) in der Breitenrichtung (W) des Beinabschnitts in gleichmäßigen Abständen und in einer geraden Linie angeordnet ist.

## Revendications

1. Semelle (5) d'une prothèse de jambe athlétique (1), la prothèse de jambe athlétique (1) présentant une partie jambe (2) en forme de ressort à lames s'étendant jusqu'à un côté d'un orteil (T) via au moins une partie courbée (3), la semelle (5) étant configurée pour être fixée à une région de contact avec le sol (4s) de la prothèse de jambe athlétique (1), la région de contact avec le sol (4s) s'étendant à partir de l'orteil (T) vers un côté arrière dans une direction avant-arrière (Y) de la partie jambe selon un premier arc (X1) et un second arc (X2) et étant convexe à un côté de contact avec le sol, dans laquelle

   la semelle (5) inclut une surface inférieure (5s) présentant une forme conforme à une forme d'extension de la région de contact avec le sol (4s), et
   la surface inférieure (5s) inclut au moins une lamelle (13) s'étendant linéairement au niveau d'une première partie (Q) correspondant au premier arc (X1) qui continue à un rayon de courbure constant à partir d'un orteil de la surface inférieure (5s), et la au moins une lamelle (13) présente une largeur de rainure inférieure à 1 mm, n'est pas ouverte au niveau d'un bord d'extrémité de la semelle (5) et présente deux extrémités qui se terminent dans la première partie (Q) de la surface inférieure (5s),
   dans laquelle la au moins une lamelle (13) comprend une pluralité de lamelles (13),
   dans laquelle la pluralité de lamelles (13) incluent au moins une paire (130) de deux lamelles à symétrie linéaire (13A, 13B) dans une relation de symétrie linéaire avec une ligne le long d'une direction avant-arrière (Y) de la partie jambe (2) étant définie sur un axe de symétrie,
   dans laquelle, dans la paire de lamelles à symétrie linéaire (130), chaque lamelle (13A, 13B) s'étend dans une direction inclinée par rapport à l'axe de symétrie,
   dans laquelle la surface inférieure (5s) présente, au niveau de la première partie (Q) de la surface inférieure (5s), un réseau de paires de lamelles (130) dans lequel une pluralité de paires de lamelles (130) à symétrie linéaire sont agencées dans la direction de largeur (W) de la partie jambe et, au niveau d'une seconde partie de la surface inférieure (5s) s'étendant du côté de la partie courbée (3) de la première partie (Q) de la surface inférieure (5s), une pluralité de parties planes de direction de largeur (10, 11, 12, 14) s'étendant pour être de forme ondulée dans la direction de largeur (W) de la partie jambe définie par une pluralité de rainures de direction de largeur s'étendant dans la direction de largeur (W) de la partie jambe, et

   un tel agencement mutuel est obtenu de sorte qu'une phase d'agencement de chaque paire de lamelles à symétrie linéaire (130) est synchronisée avec une phase d'ondes de chaque partie plane de direction de largeur (10, 11, 12, 14).

2. Semelle (5) de la jambe prothétique athlétique (1) selon la revendication 1, dans laquelle la lamelle (13) est linéaire sans aucune partie incurvée.

3. Semelle (5) de la jambe prothétique athlétique (1) selon la revendication 1 ou la revendication 2, dans laquelle un angle d'inclinaison 0 par rapport à l'axe de symétrie de chaque lamelle (13) est de 20° à 50°.

4. Semelle (5) de la jambe prothétique athlétique (1) selon l'une quelconque des revendications 1 à 3, dans laquelle, dans la paire de lamelles à symétrie linéaire (130), chaque lamelle (13A, 13B) s'étend dans une direction convergente par rapport à l'axe de symétrie, vers le côté de l'orteil.

5. Semelle (5) de la jambe prothétique athlétique (1) selon l'une quelconque des revendications 1 à 4, dans laquelle le réseau de paires de lamelles à symétrie linéaire (130) est agencé dans la direction de largeur (W) de la partie jambe à intervalles réguliers et en ligne droite.

# FIG. 1

# FIG. 2A

# FIG. 2B

# FIG. 2C

# FIG. 2D

# FIG. 3

## FIG. 4A

13C

W

Y

## FIG. 4B

13D

W

Y

## FIG. 4C

13E

W

Y

# FIG. 5

EP 3 782 589 B1

# FIG. 6

# FIG. 7

# FIG. 8

# FIG. 9A

FIG. 9B

EP 3 782 589 B1

## FIG. 10A

## FIG. 10B

## FIG. 10C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016150189 A
- US 8535390 B1
- US 2016045337 A1
- US 2017281371 A1
- US 9629415 B2